# EUROPÄISCHE PATENTSCHRIFT

(11) **EP 0 792 139 B1**
(45) Veröffentlichungstag und Bekanntmachung des Hinweises auf die Patenterteilung: **23.06.1999**
(21) Anmeldenummer: 95937879.5
(22) Anmeldetag: 08.11.1995
(51) Int. Cl.: A61K 7/13

(54) **OXIDATIONSFÄRBEMITTEL**
OXIDATION DYES
COLORANTS D'OXYDATION

(30) Priorität: 17.11.1994 DE 4440957
(43) Veröffentlichungstag der Anmeldung: 03.09.1997
(73) Patentinhaber: Henkel Kommanditgesellschaft auf Aktien, 40191 Düsseldorf (DE)
(72) Erfinder: ROSE, David, 40723 Hilden (DE); HÖFFKES, Horst, 40595 Düsseldorf (DE); MEINIGKE, Bernd, 51399 Burscheid (DE)
(86) Internationale Anmeldenummer: EP9504385
(87) Internationale Veröffentlichungsnummer: WO9615765

(56) Entgegenhaltungen:
- EP-A- 0 007 537
- EP-A- 0 039 030
- EP-A- 0 063 736
- EP-A- 0 400 330
- WO-A-88/00823
- DE-A- 3 627 398
- DE-A- 3 834 142
- DE-A- 4 122 748
- GB-A- 2 168 727

## Beschreibung

Die Erfindung betrifft Oxidationsfärbemittel zum Färben von Keratinfasern, die eine spezielle Entwickler/Kuppler-Kombination enthalten.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol, 2-Hydroxy-4,5,6-triaminopyrimidin, 2,4-Dihydroxy-5,6-diaminopyrimidin und 2,5,6-Triaminohydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol und 2-Methylresorcin.

Eine bestimmte Entwicklerkomponente kann durch Kombination mit unterschiedlichen Kupplern auch sehr unterschiedliche Farbnuancen bilden. Trotzdem gelingt es oft nicht, mit Hilfe einer einzigen Entwicklerkomponente zu der Vielzahl natürlicher Farbnuancen zu kommen. In der Praxis ist daher meist eine Kombination verschiedener Entwicklerkomponenten und Kupplerkomponenten erforderlich, um eine einzige, natürlich wirkende Färbung zu erhalten. Es besteht daher ständig Bedarf an neuen, verbesserten Kuppler/Entwickler-Kombinationen. Dies trifft insbesondere auch auf den Blau-Bereich zu, wo die gängigen Farbstoffe häufig noch nicht ganz befriedigende Egalisiervermögen und Kaltwell- und Waschechtheiten aufweisen.

Es war daher die Aufgabe der vorliegenden Erfindung, neue Entwickler-Kuppler-Kombinationen im Blau-Bereich zu finden, die die an Oxidationsfarbstoffvorprodukte zu stellenden Anforderungen in besonderem Maße erfüllen.

Überraschenderweise wurde nun gefunden, daß eine spezielle Kombination aus einer bekannten Kupplerkomponente und einer bekannten Entwicklerkomponente zu dunkelblauen Färbungen hoher Brillanz führt, die sich zusätzlich durch gute Reibechtheit auszeichnen.

Gegenstand der vorliegenden Erfindung sind Oxidationsfärbemittel zum Färben von Keratinfasern enthaltend Kupplerkomponenten und Entwicklerkomponenten in einem wasserhaltigen Träger, dadurch gekennzeichnet, daß 2-(2,5-Diaminophenyl)-ethanol oder dessen Salz mit einer anorganischen oder organischen Säure als Entwicklerkomponente und 2-Chlor-6-methyl-3-aminophenol oder dessen Salz mit einer anorganischen oder organischen Säure als Kupplerkomponente enthalten ist.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäße Entwicklerkomponente ist bereits aus der deutschen Offenlegungsschrift 28 31 847 bekannt, auf die bezüglich der Herstellung dieser Verbindung ausdrücklich Bezug genommen wird.

Die erfindungsgemäße Kupplerkomponente ist aus der deutschen Offenlegungsschrift 30 16 008 bekannt als Kupplerkomponente insbesondere für intensive Blautöne. Auch auf diese Druckschrift wird hiermit ausdrücklich Bezug genommen.

Keiner dieser Druckschriften ist aber irgendein Hinweis auf die erfindungsgemäße Kombination oder gar auf deren überaus vorteilhafte Eigenschaften zu entnehmen.

Die erfindungsgemäßen Entwickler- und Kupplerkomponenten können sowohl als freie Basen als auch in Form ihrer anorganischen oder organischen Salze, z.B. der Hydrochloride oder Hydrobromide, eingesetzt werden.

Die erfindungsgemäßen Haarfärbemittel enthalten sowohl die Entwicklerkomponenten als auch die Kupplerkomponenten bevorzugt in einer Menge von 0,01 bis 20 Gew.-%, vorzugsweise 0,5 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Neben der erfindungsgemäßen Kuppler-/Entwickler-Kombination können die Haarfärbemittel gewünschtenfalls weitere Kuppler- und/oder Entwicklerkomponenten enthalten, um spezielle Farbnuancen zu erhalten. Geeignete Verbindungen wurden bei der Diskussion des Standes der Technik bereits genannt.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Nitroblau, Disperse Blue 3, Basic Blue 99, HC Violet 1, Disperse Violet 1, Disperse Violet 4, Disperse Black 9, Basic Brown 16, Pikraminsäure und Rodol 9 R, bekannten Verbindungen, in einer Menge von 0,01 bis 20 Gew.-%, bezogen auf das gesamte Oxidationshaarfärbemittel.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen-glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Ein Beispiel für ein als kationisches Tensid einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Mengen vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Das nachfolgende Beispiel soll den Erfindungsgegenstand näher erläutern.

### Beispiel

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol^{R}techn.¹ | 4,0 |
| Texapon^{R}N 28² | 40,0 |
| Dehyton^{R}K³ | 25,0 |
| Eumulgin^{R}B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO- (ca. 30 % Aktivsubstanz; CTFA-Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrainten aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Die Ausfärbung mit der erfindungsgemäßen Kuppler-/Entwickler-Kombination war dunkelblau. Die Reibechtheit der gefärbten Haare war groß.

Weiterhin wurden die Ausfärbungen von folgenden Haarfärbecremeemulsionen gemäß oben genannter Basisrezeptur untersucht:

| Entwicklerkomponente: | B1 | B2 | B3 |
|---|---|---|---|
| 2-(2,5-Diaminophenyl)ethanol | 7,5 mmol | 7,5 mmol | 7,5 mmol |

| Kupplerkomponente: | | | |
|---|---|---|---|
| 2-Chlor-6-methyl-3-aminophenol | 7,5 mmol | 7,5 mmol | 7,5 mmol |

| weitere Komponenten: | | | |
|---|---|---|---|
| 5-Amino-2-methyl-phenol | 0,025 | - | 0,05 |
| 4-(N-2-Hydroxyethyl)-3-nitro-anilin | 0,25 | - | - |
| 2-Amino-6-chloro-4-nitrophenol | - | 0,25 | - |
| Resorcin | - | 0,17 | - |
| 2,4,5,6-Tetraaminopyrimidin | - | - | 0,13 |
| Ausfärbung | Magenta | Intensiv-braun | violett |

## Patentansprüche

1. Oxidationsfärbemittel zum Färben von Keratinfasern enthaltend Kupplerkomponenten und Entwicklerkomponenten in einem wasserhaltigen Träger, dadurch gekennzeichnet, daß 2-(2,5-Diaminophenyl)-ethanol oder dessen Salz mit einer anorganischen oder organischen Säure als Entwicklerkomponente und 2-Chlor-6-methyl-3-aminophenol oder dessen Salz mit einer anorganischen oder organischen Säure als Kupplerkomponente enthalten ist.

2. Oxidationsfärbemittel nach Anspruch 1, dadurch gekennzeichnet, daß Entwicklerkomponenten in einer Menge von 0,01 bis 20 Gew.-%, vorzugsweise 0,5 bis 5 Gew.-%, und Kupplerkomponenten in einer Menge von 0,01 bis 20 Gew.-%, vorzugsweise 0,5 bis 5 Gew.-%, jeweils bezogen auf das gesamte Oxidationsfärbemittel, enthalten sind.

3. Oxidationsfärbemittel nach Anspruch 1 oder 2, dadurch gekennzeichnet, daß mindestens eine weitere Entwicklerkomponente und/oder mindestens eine weitere Kupplerkomponente enthalten ist.

4. Oxidationsfärbemittel nach einem der Ansprüche 1 bis 3, dadurch gekennzeichnet, daß mindestens ein direktziehender Farbstoff enthalten ist.

## Claims

1. Oxidation colorants for coloring keratin fibers containing primary intermediates and secondary intermediates in a water-containing carrier, characterized in that 2-(2,5-diaminophenyl)-ethanol or a salt thereof with an inorganic or organic acid is present as the developer component while 2-chloro-6-methyl-3-aminophenol or a salt thereof with an inorganic or organic acid is present as the secondary intermediate.

2. Oxidation colorants as claimed in claim 1, characterized in that the primary intermediates are present in a quantity of 0.01 to 20% by weight and preferably in a quantity of 0.5 to 5% by weight while the secondary intermediates are present in a quantity of 0.01 to 20% by weight and preferably in a quantity of 0.5 to 5% by weight, based on the oxidation colorant as a whole.

3. Oxidation colorants as claimed in claim 1 or 2, characterized in that at least one other primary intermediate and/or at least one other secondary intermediate is present.

4. An oxidation colorant as claimed in any of claims 1 to 3, characterized in that at least one substantive dye is present.

## Revendications

1. Colorant d'oxydation pour la coloration des fibres de kératine, contenant des composants d'agent de couplage et des composants de révélateur dans un support contenant de l'eau,
caractérisé en ce que
le 2-(2,5-diaminophényl)éthanol ou son sel avec un acide minéral ou organique code composant révélateur et du 2-chloro-6-méthyl-3-aminophénol ou son sel avec un acide minéral ou organique code composant d'agent de couplage sont contenus.

2. Colorant d'oxydation selon la revendication 1,
caractérisé en ce que
les composants de révélateur sont contenus en une quantité allant de 0,01 à 20 % en poids -de préférence de 0,5 à 5 % en poids- et les composants d'agent de couplage en une quantité allant de 0,01 à 20 % en poids -de préférence de 0,5 à 5 % en poids- à chaque fois rapporté au colorant d'oxydation total.

3. Colorant d'oxydation selon la revendication 1 ou la revendication 2,
caractérisé en ce qu'
au moins un autre composant de révélateur et/ou au moins un autre composant d'agent de couplage est contenu.

4. Colorant d'oxydation selon l'une des revendications 1 à 3,
caractérisé en ce qu'
au moins un colorant direct est contenu.
